# EUROPÄISCHE PATENTSCHRIFT

(11) **EP 2 468 923 B1**
(45) Veröffentlichungstag und Bekanntmachung des Hinweises auf die Patenterteilung: **18.09.2019**
(21) Anmeldenummer: 11009583.3
(22) Anmeldetag: 05.12.2011
(51) Int. Cl.: C23C 24/00, A61L 27/32, B05D 1/28, A61L 27/54

(54) **Beschichtungsverfahren und Beschichtungsvorrichtung**
Surface coating method and coating device
Procédé de revêtement et dispositif de revêtement

(30) Priorität: 23.12.2010 DE 102010055561
(43) Veröffentlichungstag der Anmeldung: 27.06.2012
(73) Patentinhaber: Heraeus Medical GmbH, 61273 Wehrheim (DE)
(72) Erfinder: Vogt, Sebastian, Dr., 99092 Erfurt (DE); Büchner, Hubert, Dr., 90491 Nürnberg (DE)
(74) Vertreter: Heraeus IP

(56) Entgegenhaltungen:
- EP-A2- 0 306 212
- EP-A2- 2 392 360
- WO-A1-2005/042045
- WO-A2-2009/062671
- US-A- 4 532 929
- US-A1- 2006 188 541
- US-A1- 2006 251 824
- US-B1- 6 368 658
- GROOT DE K ET AL: "CALCIUM PHOSPHATE COATINGS FOR MEDICAL IMPLANTS", PROCEEDINGS OF THE INSTITUTION OF MECHANICAL ENGINEERS.JOURNAL OF ENGINEERING IN MEDICINE. PART H, MECHANICAL ENGINEERING PUBLICATIONS LTD, LONDON, GB, Bd. 212, Nr. H02, 1. Januar 1998 (1998-01-01), Seiten 137-147, XP000823765, ISSN: 0954-4119, DOI: 10.1243/0954411981533917

## Beschreibung

Die Erfindung betrifft eine Vorrichtung zur zumindest bereichsweisen Beschichtung eines medizinischen Implantats, vorzugsweise eines künstlichen Gelenks oder einer Fixierung für ein Gelenk.

Die Erfindung betrifft auch ein Verfahren zur zumindest bereichsweisen Beschichtung eines medizinischen Implantats unter Verwendung einer solchen Vorrichtung.

Die Beschichtung von medizinischen Implantaten mit pharmazeutischen Wirkstoffen hat in den letzten Jahren zunehmend Beachtung gefunden. Eine zentrale Anwendung von Beschichtungsverfahren liegt dabei im antibiotischen Schutz der Oberfläche von Implantatmaterialien. Eine weitere wichtige Anwendung ist in der Verbesserung der Oberflächenkompatibilität von nicht zu zementierenden medizinischen Implantaten zum besseren Anwachsen von Knochenmaterial zu sehen.

Die Implantation von Gelenkendoprothesen und auch von Osteosynthesematerialien ist immer mit einer gewissen Gefahr einer mikrobiellen Kontamination verbunden. Wenn es mikrobiellen Keimen gelingt, sich an der Implantatoberfläche anzusiedeln, kann es zur Ausbildung einer post-operativen Osteitis/Osteomyelitis kommen. Die Osteitis/Osteomyelitis stellt eine schwerwiegende Komplikation für den Patienten dar, die zusätzlich mit erheblichen Kosten verbunden ist.

Seit Jahrzehenten wird mit klinischem Erfolg bei zementierten Gelenkendoprothesen mit Gentamicin dotierter PMMA-Knochenzement verwendet. Das im Knochenzement enthaltende Breitbandantibiotikum Gentamicin schützt die Oberfläche des Knochenzementes wirksam gegen bakterielle Infektionen.

Bei nicht-zementierten Gelenkendoprothesen und bei Osteosynthesematerialien wurde eine Reihe von Lösungswegen vorgeschlagen, um ebenfalls einen lokalen antibiotischen Schutz der Implantatoberflächen zu erreichen.

So wurde in mehreren Patentdokumenten die Verwendung von in Wasser gering löslichen Antibiotika-Salzen beschrieben. Exemplarisch seien dafür die EP 0 623 349 A1, EP 1 470 829 A1, EP 1 374 923 A2, DE 101 42 465 A1 und DE 44 04 018 A1 genannt. Diese in Wasser gering löslichen Salze lösen sich unter Freisetzung der enthaltenen Antibiotika durch Einwirkung von Körperflüssigkeiten auf. Vorteilhaft ist die protrahierte Wirkstofffreisetzung. Nachteilig ist jedoch die aufwendige Herstellung dieser Salze.

Alternativ dazu ist es auch möglich, in Wasser lösliche Antibiotika-Salze zu verwenden. Das Problem liegt dabei darin, das Antibiotikum auf der Implantatoberfläche zu fixieren.

Die Mehrzahl der bisherigen beschriebenen Beschichtungen ist vorzugsweise für die Fertigung von beschichteten Implantaten unter industriellen Bedingungen bestimmt. Das bedeutet, dass die industrielle Beschichtung dieser Implantate nur mit wenigen für die Großanwendung relevanten Wirkstoffen erfolgen kann, um eine wirtschaftliche industrielle Fertigung mit entsprechenden hohen Stückzahlen gewährleisten zu können.

Insbesondere bei antibiotischen Beschichtungen ist es jedoch im Hinblick auf die sich zunehmend problematisch gestaltende Resistenzsituation und dem dabei vermehrten Auftreten von multiresistenten Keimen, wie MRSA und MRSE von Interesse, genau auf die jeweiligen Keime abgestimmte Antibiotika oder Antibiotika-Kombinationen zur Beschichtung von Revisionsprothesen im Zuge des einzeitigen oder zweizeitigen septischen Gelenkprothesenwechsels einzusetzen, um einen initialen antibiotischen Schutz der Implantatoberflächen zu gewährleisten.

Nachteilig ist hieran, dass die Verfahren zur Beschichtung der medizinischen Implantate relativ aufwendig sind. Eine variable kurzfristige Anwendung ist nicht möglich. Es müssen für verschiedene Situationen verschiedene beschichtete medizinische Implantate bereitgehalten werden, um den Bedürfnissen der unterschiedlichen Patienten gerecht zu werden. Dies erfordert eine umfangreiche Lagerhaltung und verhindert ungewöhnliche Mischungen für spezielle Fälle.

Nicht zementierte Gelenkendoprothesen sind im Allgemeinen aus Titanlegierungen gefertigt und besitzen meistens eine aufgeraute (beispielsweise durch Sandstrahlen) oder eine strukturierte poröse Oberfläche, um ein Anwachsen des Knochengewebes zu verbessern. Die mechanische Stabilität und Integrität der bisher eingesetzten Legierungen ist gewährleitstet. Deshalb wurde versucht, die Kompatibilität der Implantatoberflächen gegenüber dem Knochengewebe zu verbessern. Die mineralische Phase des humanen Knochengewebes wird durch einen Carbonat-Apatit/Hydroxylapatit gebildet. Deshalb liegt in der Entwicklung von Calciumphosphat-Schichten der Hauptschwerpunkt zur Verbesserung der Oberflächenkompatibilität von medizinischen Implantaten.

Im Bereich des Knochengewebekontakts (Hüft-, Knie-, Schulter-Gelenkendoprothesen) wurde mit unterschiedlichsten Verfahren (thermische Spritzverfahren, elektrochemische Abscheidung, Sol-Gel-Technologien, Ionenstrahlsputtern, Laserablation) versucht, eine Verbesserung der Oberflächenkompatibilität zu erreichen. Es konnten sich bisher industriell nur das Plasmaspritzverfahren (De Groot et al.: Plasma-sprayed coatings of calcium phosphate. CRC Press, Boca Raton, Ann Arbor, Boston, 1990; De Groot et al.: Chemistry of calcium phosphate bioceramics. CRC Handbook of bioactive ceramics, 2, 1996, 3-16.; WO 2009/062671 A2) und die elektrochemische Abscheidung von Calciumphosphat-Schichten (Ban und Maruno: Morphology and microstructure of electrochemically deposited calcium phosphates in a modified simulated body fluid. Biomaterials, 19, 1998, 1245-1253.; DE 44 31 862 A1; WO 2009/147045 A1; CN 101485901 A; CN 101406711 A; WO 2007/147246 A1; US 2006/134160 A1; WO 2004/098436 A2; WO 2004/024201 A2; EP 1 264 606 A1; EP 0 232 791 A2) durchsetzen. In den Druckschriften US 2002/110541 A1, US 5,807,567 A, US 2002/197315 A1, US 6,652,887 B1, US 5,756,127 A und US 5,614,206 A wurden Knochenersatzmaterialien beschrieben, die im Wesentlichen aus einer Mischung aus α- und β-Calciumphosphat bestehen und als "Drug-Delivery"-Systeme für pharmazeutische Wirkstoffe verwendet werden sollen.

Klinische Langzeituntersuchungen zeigen jedoch, dass die als langzeitstabil geltenden plasmagespritzten Calciumphosphat-Schichten im biologischen Umgebungsmilieu einer partiellen Degradation unterliegen. Es kommt einerseits zu Phasenänderungen an der Grenzfläche zum Knochengewebe, andererseits führt dieser Prozess zum Abkapseln und/oder Abplatzen von vorwiegend kristallinen Schichtbestandteilen und damit zu störenden Partikeln.

Nachteilig ist ferner bei allen bisher üblichen elektrochemischen Abscheidungsverfahren, dass ein erheblicher apparativer und zeitlicher Aufwand notwendig ist, um diese Calciumphosphat-Schichten auf die Gelenkendoprothesen aufzubringen.

US2006/0188541 offenbart eine Beschichtung für Implantate per Hand durch einen Schwamm, Wischtuch oder Spatula.

Die Aufgabe der Erfindung besteht also darin, die Nachteile des Stands der Technik zu überwinden. Insbesondere soll ein einfaches und leicht anzuwendendes Verfahren und eine Vorrichtung hierfür bereitgestellt werden, mit denen eine medizinische Prothese beschichtet werden kann, ohne dass dadurch der Ablauf einer Operation (OP) gestört wird. Es sollen möglichst viele verschiedene medizinische Implantate mit dem Verfahren und der Vorrichtung beschichtet werden können. Auch sollen das Verfahren und die Vorrichtung variabel einsetzbar sein, so dass sie an die medizinischen Notwendigkeiten, insbesondere an eine für den Patienten geeignete Medikamentierung anpassbar sind. Der in Operationssälen gebotenen Reinheit soll ebenfalls Rechnung getragen werden.

Die Aufgabe der Erfindung besteht ferner darin, ein möglichst einfaches Beschichtungsverfahren zu entwickeln, das vom OP-Personal während einer OP mit geringstem Zeitaufwand zum Beschichten von unterschiedlichsten Implantaten, die von beliebigen Herstellern produziert sein können, mit pharmazeutischen Zubereitungen eingesetzt werden kann. Weiterhin ist es eine Aufgabe der Erfindung, eine einfache Beschichtungsvorrichtung zu entwickeln, die es dem OP-Personal erlaubt, unter OP-Bedingungen Implantate mit geringstem Aufwand zu beschichten. Die Vorrichtung soll weiterhin so gestaltet sein, dass möglichst kein überschüssiges Material zur Herstellung der Beschichtung den OP-Bereich kontaminieren kann. Eine weitere Aufgabe besteht darin, dass die Vorrichtung besonders zur Beschichtung von nicht-zementierten Gelenkendoprothesen und Osteosynthesematerialien geeignet sein soll.

Die Aufgabe der Erfindung wird durch die Ansprüche 1 und 10 gelöst.

Vorzugsweise erfolgt das Kontaktieren derart, dass die zu beschichtende Oberfläche des medizinischen Implantats in ein Pulver getaucht oder auf ein solches Pulver gedrückt wird, wobei durch das Eintauchen oder Aufdrücken Pulver auf die Oberfläche des medizinischen Implantats übertragen wird und die zu beschichtende Oberfläche aus dem Pulver herausgezogen wird oder der Druck gelöst wird, wobei wenigstens ein Teil des Pulvers auf der zu beschichtenden Oberfläche haftet.

Erfindungsgemäße Verfahren erfolgen vor dem Einsetzen der medizinischen Implantate. Die Verfahren finden also "ex vivo" statt.

Unter einer pharmazeutisch aktiven Substanz sind erfindungsgemäß pharmazeutisch wirksame Mittel und Mittel zu verstehen, die pharmakologisch wirken, sowie solche, die eine pharmakologische Wirkung unterstützen oder sonst in irgendeiner Weise die Selbstheilungskräfte des Körpers unterstützen. Beispiele sind hierfür Antibiotika, organische Antiseptika, Kupfersalze, Kupferoxid, Galliumsalze, Strontiumsalze, Lithiumsalze, Silbersalze, Silberoxid, Bisphosphonate, Wachstumsfaktoren, Steroidhormone, nichtsteroidale Hormone, Hämostyptika, Antiphlogistika, Plasmide, Cosmide, lineare DNA und deren Mischungen.

Gemäß einer bevorzugten Ausführungsform erfolgt das Kontaktieren mit dem Pulver durch ein Eintauchen in das Pulver oder ein Aufdrücken auf das Pulver. Unter einem Eintauchen ist erfindungsgemäß nicht nur ein Eintauchen in einer großen Menge des Pulvers zu verstehen. Ein erfindungsgemäßes Aufdrücken liegt beispielsweise dann vor, wenn das Pulver an einer Oberfläche angeordnet ist, die ungefähr wie das zu beschichtende medizinische Implantat geformt ist, so dass nur eine dünne Schicht des Pulvers bereitgestellt wird. Ein Anpressen des medizinischen Implantats führt dann zu einer fast vollständigen Übertragung des Pulvers auf die Oberfläche des medizinischen Implantats.

Erfindungsgemäß ist auch, dass das zu beschichtende Implantat gegebenenfalls mehrfach in die Vorrichtung eingeführt und herausgezogen wird.

Ferner kann vorgesehen sein, dass das zu beschichtende medizinische Implantat aus nicht-zementierten Hüftgelenkendoprothesen, Schultergelenkendoprothesen, Ellbogenendoprothesen, Marknägeln und Osteosyntheseplatten ausgewählt wird.

Gemäß einer besonders vorteilhaften Ausgestaltung der Erfindung kann vorgesehen sein, dass die zu beschichtende Oberfläche des medizinischen Implantats vor dem Kontaktieren mit dem Pulver, vorzugsweise vor dem Eintauchen in das Pulver oder dem Aufdrücken auf das Pulver, mit einer Flüssigkeit kontaktiert wird, vorzugsweise in eine Flüssigkeit getaucht oder mit einer Flüssigkeit bestrichen oder benetzt wird. Auf einem mit einer Flüssigkeit benetzten medizinischen Implantat haftet das Pulver gegebenenfalls besser als auf einem trockenen medizinischen Implantat. Zudem kann die Flüssigkeit ebenfalls eine pharmazeutisch aktive Substanz umfassen.

Erfindungsgemäß kann dabei auch vorgesehen sein, dass die Flüssigkeit eine wässrige Lösung eines Antibiotikums umfasst, bevorzugt eine wässrige Gentamicinsulfat-Lösung mit einem Gentamicinsulfat-Gehalt von 10,0 bis 88,0 Gewichtsprozent verwendet wird, wobei ganz besonders eine Gentamicinsulfat-Lösung mit einem Gentamicinsulfat-Gehalt von 75,0 bis 80,0 Gewichtsprozent bevorzugt verwendet wird. Diese Gentamicinsulfat-Lösung hat eine ölig-viskose Konsistenz und haftet sehr gut auf Metalloberflächen.

Es kann dabei ferner vorgesehen sein, dass pharmazeutisch übliche Stabilisatoren in den Gentamicinsulfat-Lösungen enthalten sind. Diese verbessern die Haltbarkeit und damit die Verwendbarkeit der aufzutragenden Flüssigkeit.

Erfindungsgemäß kann auch die Verwendung von anderen Aminoglykosid-Antibiotika-Lösungen, wie wässrige Lösungen des Tobramycinsulfats, des Amikacinsulfats, des Netilmicinsulfats und des Sisomycinsulfats als Flüssigkeit oder Flüssigkeitsbestandteile vorgesehen sein. Es ist auch möglich, wässrige Lösungen des Vancomycins, des Dalbavancins, des Ramoplanins, des Daptomycins, des Moxifloxacins, des Clindamycins und des Lincomycins einzusetzen.

Ferner kann im Rahmen der Erfindung die Verwendung von Kombinationen von Lösungen verschiedener Antibiotika als Flüssigkeit vorgesehen sein. Beispielhaft sind hierbei die Zweifachkombinationen von Gentamicinsulfat mit Vancomycinhydrochlorid, die Zweifachkombination von Daptomycin mit Gentamicinsulfat und die Zweifachkombination von Gentamicinsulfat mit Clindamycin sowie die Dreifachkombination Gentamicinsulfat mit Vancomycinhydrochlorid und Clindamycinhydrochlorid.

Des Weiteren kann vorgesehen sein, dass als Flüssigkeit Antiseptika-Lösungen verwendet werden, insbesondere Lösungen des Chlorhexidindigluconats, des Octenidindihydrochlorids und des Polyhexanids.

Erfindungsgemäße Verfahren zeichnen sich dadurch aus, dass das medizinische Implantat über ein elastisch deformierbares Übertragungsmittel gestrichen wird, wobei beim Überstreichen des Übertragungsmittels eine Flüssigkeit umfassend zumindest eine pharmazeutisch aktive Substanz vom Übertragungsmittel auf die zu beschichtenden Oberfläche des medizinischen Implantats übertragen wird. Durch die Verwendung eines elastisch deformierbaren Übertragungsmittels wird erreicht, dass die durch das Übertragungsmittel aufgetragene Flüssigkeit auch auf einem unregelmäßig geformten medizinischen Implantat großflächig aufgetragen werden kann. Besonders bevorzugt ist das Übertragungsmittel auch porös, wobei die Flüssigkeit in den Poren des Übertragungsmittels gespeichert wird. Das Übertragungsmittel ist Oberhalb des Pulvers angeordnet werden, ohne dass die Flüssigkeit in das Pulver tropft. Besonders in Kombination mit einer Membran zum Abdecken des Pulvers ist dies vorteilhaft.

Nach Anspruch 10 wird das Pulver in einem Behälter mit einer Öffnung bereitgestellt, wobei das medizinische Implantat zur Beschichtung der zu beschichtenden Oberfläche durch die Öffnung eingeführt wird.

Dabei wird das medizinische Implantat vor dem Kontaktieren mit dem Pulver, vorzugsweise vor dem Eintauchen in das Pulver oder dem Aufdrücken auf das Pulver, in den Behälter eingeführt, in dem sich das Pulver befindet und nach der Übertragung des Pulvers auf das medizinische Implantat aus dem Behälter herausgezogen wird. Durch beide Maßnahmen kann das Verfahren leicht an unterschiedlichen Orten eingesetzt werden, da die zu verwendende Vorrichtung leicht transportabel ist.

Auch kann vorgesehen sein, dass das medizinische Implantat durch eine Membran gestoßen wird oder eine Membran geöffnet wird, bevor das medizinische Implantat mit dem Pulver kontaktiert wird, wobei die Membran das Pulver zumindest bereichsweise abdeckt, vorzugsweise die Membran das Pulver im Behälter abdichtet. Die Membran verhindert eine Kontamination des Pulvers vor der Anwendung. Durch das Durchstoßen der Membran wird sichergestellt, dass die schützende Membran erst kurz vor der Anwendung geöffnet wird. Die Membran sollte dazu derart aufgebaut sein, dass keine Fetzen oder andere Teile der Membran in das Pulver gelangen können oder am medizinischen Implantat haften.

Eine weitere Ausgestaltung des erfindungsgemäßen Verfahrens kann vorsehen, dass ein zur Behandlungssituation passendes Pulver bereitgestellt wird.

Auch kann vorgesehen sein, dass ein zur Behandlungssituation passendes Antibiotikum oder Antibiotika-Gemisch in das Pulver eingebracht wird. Durch diese beiden Maßnahmen kann auf die konkrete Behandlungssituation beim jeweiligen Patienten eingegangen werden.

Besonders bevorzugt ist, dass vorgesehen ist, dass ein Teil des übertragenen Pulvers abgestreift wird, insbesondere beim Herausziehen des medizinischen Implantats aus dem Behälter, vorzugsweise an einem dafür vorgesehenen Abstreifer. Hierdurch kann eine Kontamination der Umgebung, also insbesondere eines OP-Bereichs, mit dem Pulver und gegebenenfalls auch mit der Flüssigkeit verhindert oder zumindest reduziert werden. Dies ist vor allem bei der Verwendung von Antibiotika geboten, da so der Entwicklung resistenter Keime im OP-Bereich vorgebeugt werden kann.

Ferner kann vorgesehen sein, dass zumindest 50% der Oberfläche des medizinischen Implantats, vorzugsweise zumindest 80%, besonders bevorzugt zumindest 90% der Oberfläche des medizinischen Implantats beschichtet werden.

Um den beschichteten Bereich und die Vollständigkeit der Beschichtung sichtbar zu machen, kann erfindungsgemäß vorgesehen sein, dass das Pulver eingefärbt wird, so dass der beschichtete Bereich des medizinischen Implantats farblich kenntlich gemacht wird.

Dabei kann vorgesehen sein, dass die Vollständigkeit der Beschichtung des zu beschichtenden Bereichs anhand der Färbung geprüft wird.

Es kann ferner vorgesehen sein, dass das Verfahren so oft wiederholt wird, bis eine vollständige Beschichtung der zu beschichtenden Oberfläche des medizinischen Implantats erreicht wird. Insbesondere im Zusammenhang mit einer Färbung der Flüssigkeit und der Prüfung der Vollständigkeit der Beschichtung mit Hilfe der Färbung ist dies erfindungsgemäß vorteilhaft, um eine ausreichend beschichtetes medizinisches Implantat zu erzeugen.

Eine weitere Ausgestaltung der Erfindung sieht vor, dass das Pulver mit einem Luftstrom aufgewirbelt wird, um eine vollständige Beschichtung des medizinischen Implantats zu erreichen. Dabei kann es vorteilhaft sein, wenn der Behälter mit dem eingeschobenen Implantat zuvor geschlossen und/oder abgedichtet wird. Dies kann durch Schließen eines dafür vorgesehenen Deckels geschehen.

Ebenso kann vorgesehen sein, dass der Behälter mit dem Implantat kurz geschüttelt wird.

Die Aufgabe der Erfindung wird auch gelöst durch Anspruch 1.

Ferner kann vorgesehen sein, dass die Öffnung durch einen abziehbaren Deckel verschlossen ist. Hierdurch kann eine Verunreinigung des Inneren des Behälters vermieden werden.

Eine besonders vorteilhafte Ausgestaltung der Erfindung kann vorsehen, dass die Vorrichtung einen Abstreifer umfasst, der vorzugsweise im Bereich der Öffnung, insbesondere zwischen der Öffnung und dem Pulver, angeordnet ist.

Dabei kann vorgesehen sein, dass der Abstreifer scheibenförmig ist und mindestens einen Einschnitt umfasst, der die Oberseite und die Unterseite der Scheibe verbindet. Durch diesen mindesten einen Einschnitt kann das Implantat in die Vorrichtung eingebracht werden. Besonders vorteilhaft ist es, wenn radiale Einschnitte in dem Abstreifer ausgebildet sind. Dies macht es möglich, den gesamten äußeren Umfang der Implantate nach Beendigung der Beschichtung abzustreifen und damit überschüssige Mengen der Lösung oder Suspension von der beschichteten Implantatoberfläche zu entfernen. Weiterhin ist es dadurch möglich, die Freisetzung von Tröpfchen oder Partikeln des Pulvers und/oder der Flüssigkeit, die beim Herausziehen des Implantats aus dem Übertragungsmittel entstehen können, wirksam zurück zu halten. Damit wird die Kontamination des OPs weitgehend vermieden.

Es kann des Weiteren vorgesehen sein, dass der Abstreifer als Kegelmantel oder als Halbkugelfläche ausgebildet ist, wobei die Kegelspitze oder die Halbkugel in Richtung des Pulvers ausgerichtet ist und vorzugsweise der Kegel oder die Halbkugel mindestens einen Einschnitt enthalten, der die Oberseite und die Unterseite des Abstreifers verbindet.

Erfindungsgemäß ist über dem Pulver ein Übertragungsmittel angeordnet, mit dem eine Flüssigkeit auf das medizinische Implantat übertragbar ist, wobei die Flüssigkeit in dem Übertragungsmittel enthalten ist.

Dabei ist es vorgesehen, dass das Übertragungsmittel Poren umfasst und in den Poren des Übertragungsmittels die Flüssigkeit, vorzugsweise in Form einer Lösung und/oder Suspension enthalten ist, wobei in der Flüssigkeit eine zweite pharmazeutisch aktive Substanz enthalten ist.

Eine Weiterbildung der Erfindung sieht vor, dass das Übertragungsmittel zumindest einen Schwamm umfasst, mit der oder dem oder mit denen die Flüssigkeit auf die zu beschichtende Oberfläche des medizinischen Implantats übertragbar ist. Dadurch kann die Menge der zu verwendenden Flüssigkeit reduziert werden und ein ungewolltes Vermischen größerer Mengen der Flüssigkeit mit dem Pulver vermieden werden.

Gemäß einer besonders bevorzugten Ausgestaltung der Erfindung kann vorgesehen sein, dass das Pulver und vorzugsweise auch die Flüssigkeit Antibiotika und/oder organische Antiseptika beinhaltet, so dass die zu erzeugende Beschichtung eine pharmazeutisch wirksame Dosis beinhaltet.

Ferner kann vorgesehen sein, dass die Vorrichtung einen Vakuumanschluss umfasst, der mit einer Vakuumquelle verbindbar ist und der vorzugsweise zwischen dem Abstreifer und dem Pulver angeordnet ist. Dadurch kann zusätzlich durch Absaugen von eventuellen Pulverresten und gegebenenfalls auch Tröpfchen der Flüssigkeit sichergestellt werden, dass keine Kontamination des OPs mit pharmazeutischen Wirkstoffen erfolgt.

Erfindungsgemäß sind die Vorrichtungen mit einem Übertragungsmittel zum Auftragen einer Flüssigkeit auch vorgesehen, dass der Behälter und/oder der Abstreifer bevorzugt aus einem hydrophoben Material gefertigt sind und dass das Übertragungsmittel bevorzugt aus einem hydrophilen Material gefertigt ist. Bevorzugt sind wässrige Lösungen und/oder Suspensionen von pharmazeutischen Wirkstoffen für die Flüssigkeit. Wenn das Übertragungsmittel aus einem hydrophilen Material gefertigt ist, befinden sich wässrige Lösungen und/oder Suspensionen bevorzugt in dem porösen hydrophilen Material und nicht auf der hydrophoben Oberfläche des Behälters und des Abstreifers. Dieses Verhalten ermöglicht es, das mit wässrigen Lösungen und/oder Suspensionen vorgefüllte Beschichtungsvorrichtungen mit geringsten Volumina dieser wässrigen Lösungen oder Suspensionen auskommen und trotzdem eine sichere Beschichtung ermöglichen.

Auch kann vorgesehen sein, dass der Abstreifer aus einem biokompatiblen Elastomer, thermoplastischen Kunststoff oder auch durch eine Metallfolie oder auch aus Kompositen, die aus Metall-Elastomer-Kombinationen oder Metall-Kunststoffkombinationen gefertigt sind, besteht. Ferner kann vorgesehen sein, dass der Abstreifer als Ring ausgebildet ist, der radial zum Mittelpunkt des Behälters angeordnete Borsten enthält. Diese Borsten können aus Kunststoff gebildet sein, wobei die Borsten mechanisch so stabil und so stabil verankert sein müssen, dass ein Abbrechen oder Ablösen der Borsten möglichst unterbleibt.

Gemäß einer Weiterentwicklung der Erfindung kann auch vorgesehen sein, dass der Abstreifer in Form von drehbaren oder nichtdrehbaren Walzen und/oder auch Kugeln ausgebildet ist, die durch elastische Verbindungsmittel mit dem Behälter verbunden sind. Durch diesen Aufbau wird eine besonders einfache Abstreifbarkeit überschüssigen Pulvers und gegebenenfalls überschüssiger Flüssigkeit erreicht.

Die Vorrichtung ist erfindungsgemäß mit einem Pulver vorbefüllt, so dass das OP-Personal nur die Vorrichtung öffnen muss und anschließend sofort die Beschichtung des Implantats vornehmen kann. Vorteilhaft ist hierbei, dass der Zeitaufwand für diese Beschichtung nur im Bereich von wenigen Sekunden liegt und wertvolle OP-Zeit gespart werden kann.

Nach dem Stand der Technik ist es möglich, eine nicht befüllte Vorrichtung direkt im OP durch Einfüllen eines Pulvers und/oder Einspritzen einer Wirkstofflösung oder Wirkstoffsuspension mit einem oder mehreren pharmazeutischen Wirkstoffen auszurüsten. Dadurch ist es im Fall der antibiotischen Beschichtung möglich, entsprechend der vorliegenden Resistenzsituation eine geeignete Auswahl eines Antibiotikums oder einer Antibiotika-Kombination vorzunehmen und damit eine Antibiogramm-gerechte Beschichtung herzustellen.

Nach dem Stand der Technik ist es möglich vor der OP nicht befüllte Vorrichtungen in der jeweiligen Klinikapotheke mit geeigneten Wirkstofflösungen oder Wirkstoffsuspensionen zu befüllen, so dass während der OP die Beschichtung ohne Zeitverzug vorgenommen werden kann.

Beispiele für verwendbare pharmazeutisch aktive Substanzen sind Antibiotika, organische Antiseptika, Kupfersalze, Kupferoxid, Galliumsalze, Strontiumsalze, Lithiumsalze, Silbersalze, Silberoxid, Bisphosphonate, Wachstumsfaktoren, Steroidhormone, nichtsteroidale Hormone, Hämostyptika, Antiphlogistika, Plasmide, Cosmide, lineare DNA und deren Mischungen.

Als Flüssigkeiten können wässrige Lösung eines Antibiotikums, bevorzugt eine wässrige Gentamicinsulfat-Lösung mit einem Gentamicinsulfat-Gehalt von 10,0 bis 88,0 Gewichtsprozent vorgesehen sein, wobei ganz besonders eine Gentamicinsulfat-Lösung mit einem Gentamicinsulfat-Gehalt von 75,0 bis 80,0 Gewichtsprozent bevorzugt ist. Diese Gentamicinsulfat-Lösung hat eine ölig-viskose Konsistenz und haftet sehr gut auf Metalloberflächen. Pharmazeutisch übliche Stabilisatoren können weiterhin in den Gentamicinsulfat-Lösungen enthalten sein.

Es ist ebenfalls die Verwendung von anderen Aminoglykosid-Antibiotika-Lösungen, wie wässrige Lösungen des Tobramycinsulfats, des Amikacinsulfats, des Netilmicinsulfats und des Sisomycinsulfats. Es ist auch möglich, wässrige Lösungen des Vancomycins, des Dalbavancins, des Ramoplanins, des Daptomycins, des Moxifloxacins, des Clindamycins und/oder des Lincomycins einzusetzen. Ebenfalls im Rahmen der Erfindung ist die Verwendung von Kombinationen von Lösungen verschiedener Antibiotika. Beispielhaft sind hierbei die Zweifachkombinationen von Gentamicinsulfat mit Vancomycinhydrochlorid, die Zweifachkombination von Daptomycin mit Gentamicinsulfat und die Zweifachkombination von Gentamicinsulfat mit Clindamycin sowie die Dreifachkombination Gentamicinsulfat mit Vancomycinhydrochlorid und Clindamycinhydrochlorid. Weiterhin ist es möglich, anstelle von Antibiotika-Lösungen auch Antiseptika-Lösungen zu verwenden. Beispielhaft sind hierbei Lösungen des Chlorhexidindigluconats, des Octenidindihydrochlorids und des Polyhexanids.

Ebenfalls ist die Verwendung von Lösungen von Antibiotika und Antiseptika, die als Lösungsmittel organische Lösungsmittel oder Kombinationen von organischen Lösungsmitteln oder auch Kombinationen von organischen Lösungsmitteln und Wasser enthalten.

Es ist dadurch möglich, zum Beispiel auch in Wasser gering lösliche Antibiotika-Salze, wie Laurate, Myristate, Palmitate und Stearate zu verwenden. Daneben können auch in Wasser gering lösliche Antibiotika oder Antibiotika-Salze in Form von wässrigen Suspensionen eingesetzt werden.

Erfindungsgemäß ist, dass das Pulver als knochenwachstumsfördernde Substanz wenigstens eine Verbindung umfasst, die aus der Gruppe ausgewählt ist, die aus ß-Tricalciumphosphat, α-Tricalciumphosphat, amorphem Calciumphosphat, Tetracalciumphosphat, Octacalciumphosphat, Hydroxylapatit, Fluorapatit, Calciumsulfat-Hemihydrat, Calciumsulfat-Dihydrat, wasserfreiem Calciumsulfat, pulverförmigen Antibiotika, organischen Antiseptika, Kupfersalzen, Kupferoxid, Galliumsalzen, Strontiumsalzen, Lithiumsalzen, Silbersalzen, Silberoxid, Bisphosphonaten, Wachstumsfaktoren, Steroidhormonen, nichtsteroidalen Hormonen, Hämostyptika, Antiphlogistika, Plasmiden, Cosmiden, linearer DNA sowie deren Gemische besteht. In dem Pulver können auch Komplexbildner oder Salze enthalten sein, die mit den vom Abstreifer auf die Implantatoberfläche übertragenen pharmazeutischen Wirkstoffen in Wasser gering lösliche Komplexe oder Salze bilden. So kann zum Beispiel Teicoplanin im Pulver enthalten sein, das mit Gentamicin oder anderen kationischen Antibiotika in Wasser gering lösliche komplexe bildet. Es ist auch beispielsweise möglich, dass in dem Pulver N-Methyl-Glucamoniumsalze von Fettsäuren oder von Alkylsulfaten enthalten sind, die bei Kontakt mit wässrigen Lösungen von kationischen Antibiotika infolge eines reziproken Salzaustauschs in Wasser gering lösliche Fettsäuresalze oder Alkylsulfate der Antibiotika bilden können. Auf diese Weise ist es möglich, in Wasser gering lösliche Komplexe oder Salze von pharmazeutischen Wirkstoffen, insbesondere von Antibiotika, auf die Implantatoberfläche aufzubringen.

Besonders vorteilhaft ist es, wenn reaktive anorganische Pulver, wie amorphisiertes Calciumphosphat, Tetracalciumphosphat und Calciumsulfat-Hemihydrat, verwendet werden, die in Gegenwart von Wasser aushärten. Dadurch ist es möglich, stabile Beschichtungen auszubilden. Die Aushärtung innerhalb von wenigen Sekunden kann dabei erreicht werden, indem zum Beispiel bei Verwendung von Calciumsulfat-Hemihydrat als Pulver geringe Mengen von Calciumsulfat-Dihydrat als Keimbildner sowie Ammoniumsulfat, Natriumsulfat oder Kaliumsulfat als Beschleuniger zu dem Calciumsulfat-Hemihydrat zugegeben werden. Vorteilhaft ist weiterhin die Verwendung von ß-Tricalciumphosphat, α-Tricalciumphosphat und Tetracalciumphosphat, die durch Einwirkung von wässrigen Säuren, insbesondere von wässrigen Lösungen der Äpfelsäure, der Weinsäure und der Citronensäure innerhalb von wenigen Sekunden aushärten.

Erfindungsgemäß ist es ferner, dass die Vorrichtung als Arzneimittel oder als Medizinprodukt bereitgestellt wird.

Auch eine Kombination der erfindungsgemäßen Vorrichtung mit einem medizinischen Implantat könnte angeboten werden. Diese Kombination wird aus der Vorrichtung und dem Implantat gebildet, wobei diese Kombination eine Mindestlebensdauer von 0,1 Sekunden hat. Die Kombination entsteht besteht während des Beschichtungsprozesses.

Der Erfindung liegt die überraschende Erkenntnis zugrunde, dass ein Pulver, mit dem ein medizinisches Implantat beschichtet werden soll, auch kurz vor seiner Anwendung einfach durch Eintauchen des Implantats in das Pulver auf dem medizinischen Implantat aufgetragen werden kann. Das einfach gestaltete Verfahren und die Vorrichtung hierfür stellen dabei die Anwendbarkeit auch im OP-Bereich sicher.

Untersuchungen weisen die hohe Effizienz von vollständig degradierbaren, bioaktiven Schichten, die mittels elektrochemischer Verfahren auf metallischen Grundkörpern aufgebracht wurden, nach. Die Auswertung von tierexperimentellen und klinischen Studien führt zu der Schlussfolgerung, dass trotz der schnellen und vollständigen Degradation von hochlöslichen Calciumphosphat-Schichten ein sicheres Knochenanwachsverhalten an die Implantatoberfläche gegeben ist.

Daher können die schnell löslichen Calciumphosphat-Schichten zu klinisch guten Resultaten führen. Es ist daher keine langzeitstabile Beschichtung auf den Implantatoberflächen erforderlich.

Für einen initialen antibiotischen Schutz reicht es aus, wenn für einen Zeitraum von 24 bis 72 Stunden hinreichend hohe Antibiotikum- oder Antibiotika-Konzentrationen an den Implantatoberflächen vorhanden sind. Daher kann auch bei der lokalen Einbringung von einfachen in Wasser löslichen Antibiotika in eine Flüssigkeit ein ausreichender temporärer, lokaler, antibiotischer Schutz des medizinischen Implantats erzielt werden.

Ein feines Pulver haftet in geringem Maße aufgrund von elektronischen Wechselwirkungen auch auf reinen metallischen Implantaten. Die Staubschicht, die nach dem Eintauchen in das Pulver auf dem medizinischen Implantat zurückbleibt, kann bereits ausreichen, um eine Verbesserung des Wachstums von Knochensubstanz im Bereich des eingesetzten medizinischen Implantats zu erreichen. Eine dickere Pulverschicht kann erzeugt werden, wenn das medizinische Implantat zuvor mit einer Flüssigkeit beschichtet wird. Vorteilhafter Weise eignet sich die Flüssigkeit, die zur Beschichtung eingesetzt wird, auch ferner dazu, ein in der Flüssigkeit enthaltenes Antibiotikum oder andere wasserlösliche pharmazeutisch wirksame Substanzen auf dem medizinischen Implantat aufzutragen. Die so erzeugte Beschichtung kann eine Vielzahl verschiedener Substanzen enthalten. Da diese erst kurz vor dem Einsetzen des Implantats aufgetragen werden, können sogar Substanzen dem Pulver und der Flüssigkeit beigemischt werden, die auf längere Zeit nicht ohne weiteres mit dem Pulver oder der Flüssigkeit vermischt werden können, da sie sich gegenseitig im Laufe der Zeit beeinträchtigen.

Anstatt also das medizinische Implantat lange im Voraus bei der Herstellung zu beschichten, kann es auch unmittelbar vor dem Einsetzen beschichtet werden. Dadurch können auch relativ kurzlebige Beschichtungen verwendet werden.

Im Folgenden werden Ausführungsbeispiele der Erfindung anhand von zwei schematisch dargestellten Figuren erläutert, ohne jedoch dabei die Erfindung zu beschränken. Dabei zeigt:
Figur 1: eine schematische Querschnittansicht einer erfindungsgemäßen Vorrichtung und
Figur 2: eine schematische perspektivische Ansicht einer erfindungsgemäßen Vorrichtung.

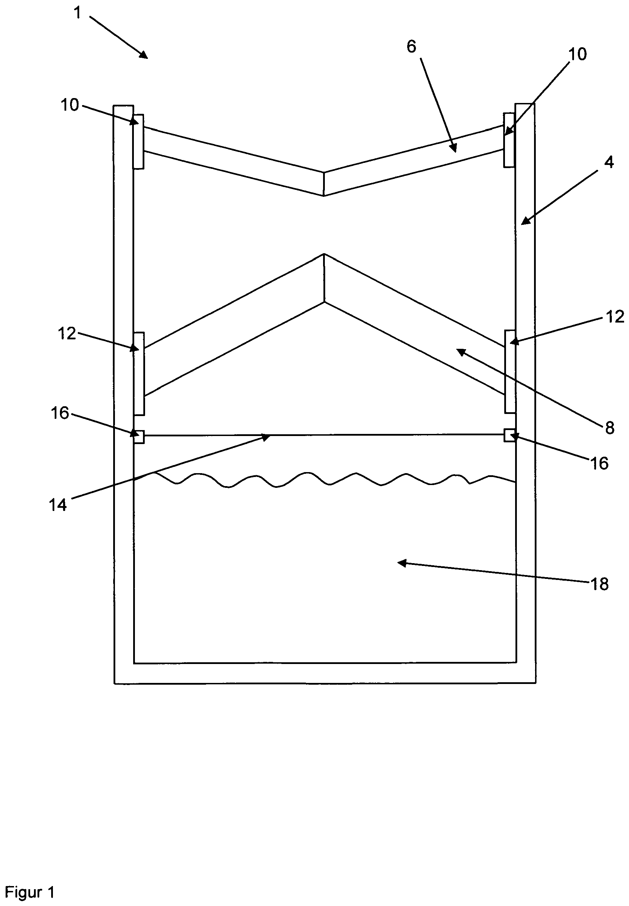

Figur 1 zeigt eine schematische Querschnittansicht einer erfindungsgemäßen Vorrichtung 1. Die Vorrichtung 1 umfasst einen Behälter 4 in Form eines oben offenen Topfs. Die Seitenwände des Behälters 4 sind zylindrisch und von gleichmäßiger Dicke. Im Bereich der Öffnung, kurz unterhalb der Öffnung ist im Inneren des Behälters 4 ein Abstreifer 6 angeordnet, der die Öffnung abschließt.

Der Boden und die Seitenwände des Behälters 4 und der Abstreifer 6 sind aus einem hydrophoben Material gefertigt oder mit einer hydrophoben Schicht beschichtet. Ausgehend von der Mitte des Abstreifers 6 ist der Abstreifer 6 in acht Richtungen geschlitzt beziehungsweise eingeschnitten.

Die acht Schlitze / Einschnitte (nicht gezeigt) reichen nicht bis zu den Seitenwänden des Behälters 4 und sollen das Einführen eines medizinischen Implantats durch den Abstreifer 6 ermöglichen. Der Abstreifer 6 hat dadurch acht flexible Segmente die beim Einführen und beim Herausnehmen, beziehungsweise beim Herausziehen des medizinischen Implantats dieses Abstreifen, das heißt die Oberfläche des Implantats überstreichen. Dadurch ist sichergestellt, dass der Abstreifer 6 im Wesentlichen die gesamte Oberfläche des medizinischen Implantats, insbesondere beim Herausziehen, überstreicht und dabei abstreift.

Im Inneren des Behälters 4 ist ein Übertragungsmittel 8 angeordnet, in dem ebenfalls Schlitze angeordnet sind. Das Übertragungsmittel 8 ist aus einem flexiblen porösen Material, wie einem Schwamm, gefertigt. Das Übertragungsmittel 8 ist mit einer wässrigen Lösung umfassend ein Antibiotikum vollgesogen. Das Material ist hydrophil. Dadurch wird sichergestellt, dass es mit einer wässrigen Flüssigkeit getränkt werden kann. Durch die hydrophoben Eigenschaften des Behälters 4 und des Abstreifers 6 befindet sich die wässrige Flüssigkeit im Übertragungsmittel 8.

Der Abstreifer 6 wird durch einen Halterungsring 10 getragen, der auf der Innenseite des Behälters 4 im Bereich der Öffnung angeordnet ist. In gleicher Weise wird das Übertragungsmittel 8 durch einen zweiten Halterungsring 12 getragen. Im zweiten Halterungsring 12 kann eine Speicherrinne vorgesehen sein, die überschüssige wässrige Flüssigkeit aufnehmen kann. Dadurch wird verhindert, dass die im Übertragungsmittel 8 enthaltene Flüssigkeit in die Bereiche unterhalb des Übertragungsmittels 8 im Behälter 4 fließt, wenn ein Druck auf das Übertragungsmittel 8 ausgeübt wird, das heißt das Übertragungsmittel ausgepresst wird. Einen Teil der im Halterungsring 12 zurückgehaltenen Flüssigkeit kann, wenn sich das Übertragungsmittel 8 entspannt, von diesem wieder aufgesaugt werden, um für eine zweite Anwendung bereitzustehen.

Unterhalb des Übertragungsmittels 8 ist im Behälter 4 eine Membran 14 angeordnet, die durch einen dritten Halterungsring 16 parallel zum Boden des Behälters 4 gehaltert wird. Die Membran 14 schließt den Behälter 4 dicht ab, so dass keine Verunreinigung von außen und keine Tropfen der in dem Übertragungsmittel 8 enthaltenen Flüssigkeit in den Bereich unterhalb der Membran 14 vordringen kann. Unterhalb der Membran 14 ist im Behälter 4 ein Pulver 18 enthalten, das eine pharmazeutisch aktive Substanz oder eine das Knochenwachstum fördernde Substanz umfasst. Bei der knochenwachstumsfördernden Substanz kann es sich beispielsweise um ein Gemisch aus Calciumsulfat-Hemihydrat, Calciumsulfat-Dihydrat und Ammoniumsulfat oder um Calciumphosphate handeln. Die Körnung des Pulvers 18 kann dabei unter 80 µm liegen, um die zu beschichtenden Implantate wirksam zu beschichten und um Verwertbarkeit der pharmazeutisch aktiven oder knochenwachstumsfördernden Substanzen im lebenden Körper sicherzustellen.

Mit der gezeigten Vorrichtung 1 kann ein erfindungsgemäßes Verfahren durchgeführt werden. Das Übertragungsmittel 8 wird mit einer wässrigen Flüssigkeit getränkt, die zumindest eine pharmazeutisch wirksame Substanz enthält, mit der ein medizinisches Implantat beschichtet werden soll. Das Übertragungsmittel 8 kann über einen Anschluss (nicht gezeigt) mit der Flüssigkeit getränkt werden. Alternativ kann durch den Abstreifer 6 hindurch das Übertragungsmittel 8 mit einer Spritze befüllt werden.

Ein medizinisches Implantat (nicht gezeigt) wird durch den Abstreifer 6 hindurch geschoben und trifft auf das Übertragungsmittel 8. Durch den Druck, der über das medizinische Implantat auf das Übertragungsmittel 8 ausgeübt wird schiebt sich zum einen das medizinische Implantat durch die dafür vorgesehenen Durchbrechungen im Übertragungsmittel 8 und zum anderen wird die in den Poren des Übertragungsmittels 8 enthaltene Flüssigkeit aus dem Übertragungsmittel 8 herausgedrückt und auf die Oberfläche des medizinischen Implantats aufgetragen.

Anschließend durchstößt das mit der Flüssigkeit beschichtete medizinische Implantat die Membran 16, die zuvor das darunter angeordnete Pulver 18 von äußeren Einflüssen bewahrt hat. Das medizinische Implantat wird dann in das Pulver 18 eingetaucht. Durch den Flüssigkeitsfilm auf der Oberfläche des medizinischen Implantats bleibt das Pulver 18 gut auf dessen Oberfläche haften.

Nachdem die Oberfläche des medizinischen Implantats mit dem Pulver 18 beschichtet wurde, wird es aus dem Behälter 4 herausgezogen. Dabei wird die beschichtete Oberfläche des medizinischen Implantats am Übertragungsmittel 8 und am Abstreifer 6 vorbeigezogen. Überschüssiges Pulver 18 und überschüssige Flüssigkeit wird dabei von der Oberfläche des medizinischen Implantats abgestreift. Das aus dem Behälter 4 herausgezogene medizinische Implantat ist dann zwar beschichtet, tropft aber nicht mehr nach und staubt auch nicht. Zudem wird durch die Neigung des Übertragungsmittels 8, das als Kegelmantel ausgeformt ist und dessen Spitze in Richtung des Abstreifers 6 weist, ein Herumspritzen der Flüssigkeit vermieden. Durch diese Maßnahmen kann verhindert werden, dass die Flüssigkeit und das Pulver 18 die Umgebung kontaminieren. Das mit dem Pulver 18 und der Flüssigkeit beschichtete medizinische Implantat ist dann bereit, um für eine Operation eingesetzt zu werden.

Die Beschichtungsvorrichtung 1 ist aus Polypropylen gefertigt, hat eine Höhe von 25 cm und einen Durchmesser von 6 cm. Der Abstreifer 6 besteht ebenfalls aus Polypropylen. Das Übertragungsmittel 8 ist ein scheibenförmiger Elastomerschwamm (Polyurethan-Schwamm) und die Membran 14 ist aus Aluminiumverbundfolie gebildet. Die Halterungsringe 10, 12, 16 des Abstreifers 6, des Übertragungsmittels 8 und der Membran 14 sind aus Polypropylen gefertigt. Die Polypropylenringe 10, 12, 16 sind im Presssitz in den Innenraum des Behälters 4 eingepresst. Der Behälter 4 ist vor der Anwendung durch eine Aluminiumverbundfolie (nicht gezeigt) keimdicht verschlossen, die die Öffnung des Behälters 4 verschließt.

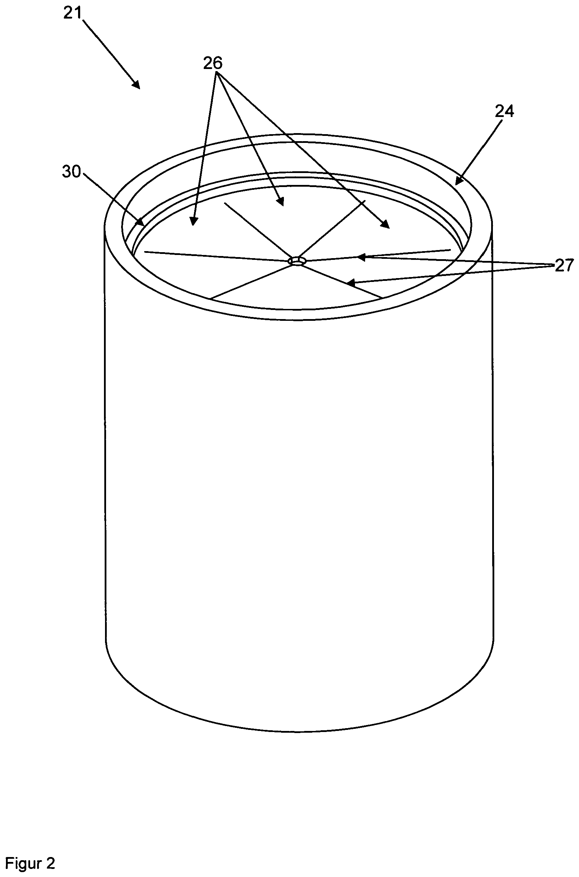

Figur 2 zeigt eine schematische perspektivische Ansicht einer zweiten erfindungsgemäßen Vorrichtung 21 für ein erfindungsgemäßes Verfahren. Die Vorrichtung 21 umfasst einen Behälter 24 und einen Abstreifer 26, der den Behälter 24 auf der Oberseite fast vollständig, bis auf eine kreisrunde Öffnung in der Mitte abschließt. Der flexible Abstreifer 26 hat sechs Schlitze 27 beziehungsweise Einschnitte 27, die die Oberseite des Abstreifers 26 mit der ins Innere des Behälters 24 gewandten Unterseite des Abstreifers 26 verbinden, so dass ein medizinisches Implantat (nicht gezeigt) durch den Abstreifer 26 in das Innere des Behälters 24 entlang der dann umgeklappten Schlitze 27 eingeführt werden kann.

Im Inneren des Behälters 24 befindet sich ein Pulver (nicht gezeigt), in das ein in den Behälter 24 eingeschobenes medizinisches Implantat eingetaucht werden kann oder auf das das medizinische Implantat aufgedrückt werden kann. Um eine Verunreinigung des Pulvers im Behälter 24 zu verhindern, kann das Pulver durch eine im Inneren des Behälters 24 angeordnete Membran (nicht gezeigt) abgedeckt sein, die mit dem medizinischen Implantat durchstoßen wird. Der Abstreifer 26 sorgt dafür, dass überschüssiges Pulver von der Oberfläche des medizinischen Implantats abgestreift wird, wenn es aus dem Behälter 24 herausgezogen wird.

Erfindungsgemäß können übliche Zweymüller-Hüftendoprothesen kurz in die mit Pulver und Flüssigkeit beziehungsweise nur mit Pulver befüllten Vorrichtungen 1, 21 bis zum Ende des Schafts hinein gesteckt und sofort wieder heraus gezogen werden. Die Zweymüller-Hüftprothesen haben dann eine Pulverbeschichtung und gegebenenfalls auch einen Film einer Flüssigkeit an der Schaftoberfläche. Wenn sowohl ein Pulver, als auch eine Flüssigkeit verwendet wird, haben die Zweymüller-Hüftprothesen dann einen weißen Belag an der Schaftoberfläche der innerhalb von maximal 30 Sekunden aushärtet und an der Oberfläche haftet. Die Hüftprothesen sind bereit zum Einsatz bei einer Operation.

Im Folgenden werden Beispiele zur Herstellung von Pulvern und von Flüssigkeiten für ein erfindungsgemäßes Verfahren und ein weiteres Beispiel für eine erfindungsgemäße Vorrichtung ausgeführt.

### Beispiel 1: Vorrichtung:

Ein durch eine Membran verschlossener Behälter ist mit einem Pulvergemisch von 150 g Calciumsulfat-Hemihydrat (Siebfraktion < 64 µm), 15,0 g Calciumsulfat-Dihydrat (Siebfraktion < 64 µm) und 1,5 g Ammoniumsulfat (Siebfraktion < 64 µm) gefüllt.

*Beispiel 2: Vorrichtung:*Der Behälter aus Beispiel 1 ist mit einem Pulvergemisch von 100 g Calciumsulfat-Hemihydrat (Siebfraktion < 64 µm), 50,0 g Calciumcarbonat (Siebfraktion < 64 µm), 15,0 g Calciumsulfat-Dihydrat (Siebfraktion < 64 µm) und 1,5 g Ammoniumsulfat gefüllt.*Beispiel 3: Erfindungsgemäße Vorrichtung:*
Der Behälter aus Beispiel 1 ist mit 150 g ß-Tricalciumphosphat (Siebfraktion < 64 µm) gefüllt.

### Beispiel 4: Vorrichtung:

Der Behälter aus Beispiel 1 ist mit 150 g α-Tricalciumphosphat (Siebfraktion < 64 µm) gefüllt.

### Beispiel 5: Vorrichtung:

Der Behälter aus Beispiel 1 ist mit 150 g α-Tetracalciumphosphat (Siebfraktion < 64 µm) gefüllt.

### Beispiel 6: Herstellung einer Beschichtungslösung mit Gentamicinsulfat:

Es wurden 16,0 g Gentamicinsulfat (Fujian Fukang Ltd.) mit 4,0 ml pyrogenfreiem sterilem Wasser bei Raumtemperatur vermischt. Nach 24 Stunden Rühren bei Raumtemperatur mit einem Magnetrührer hatte sich eine ölig-viskose gelbliche Lösung gebildet. Es ergab sich eine Beschichtungslösung mit Gentamicinsulfat als Flüssigkeit zur Beschichtung eines medizinischen Implantats.

### Beispiel 7: Herstellung einer Beschichtungslösung mit der Zweifachkombination Gentamicinsulfat und Clindamycinhydrochlorid:

Es wurden 12,0 g Gentamicinsulfat (Fujian Fukang Ltd.), 4,0 g Clindamycinhydrochlorid (Sigma-Aaldrich) mit 4,0 ml pyrogenfreiem sterilem Wasser bei Raumtemperatur vermischt. Nach 24 Stunden Rühren bei Raumtemperatur mit einem Magnetrührer hatte sich eine ölig-viskose gelbliche Lösung gebildet.

### Beispiel 8: Herstellung einer Beschichtungslösung mit der Dreifachkombination Gentamicinsulfat, Clindamycinhydrochlorid und Vancomycinhydrochlorid:

Es wurden 4,0 g Gentamicinsulfat (Fujian Fukang Ltd.), 4,0 g Clindamycinhydrochlorid (Sigma-Aaldrich) und 4,0 g Vancomycinhydrochlorid (Sigma-Aldrich) mit 8,0 ml pyrogenfreiem sterilem Wasser bei Raumtemperatur vermischt. Nach 24 Stunden Rühren bei Raumtemperatur mit einem Magnetrührer hatte sich eine viskose gelbliche Lösung gebildet.

*Beispiel 9: Herstellung einer Beschichtungslösung mit Gentamicinsulfat und Äpfelsäure:*Es wurden 100 mg Äpfelsäure und 16,0 g Gentamicinsulfat (Fujian Fukang Ltd.) mit 4,0 ml pyrogenfreiem sterilem Wasser bei Raumtemperatur vermischt. Nach 24 Stunden Rühren bei Raumtemperatur mit einem Magnetrührer hatte sich eine ölig-viskose gelbliche Lösung gebildet.

### Beispiel 10: Herstellung einer Beschichtungslösung mit Gentamicinsulfat und Citronensäure:

Es wurden 100 mg Citronensäure und 16,0 g Gentamicinsulfat (Fujian Fukang Ltd.) mit 4,0 ml pyrogenfreiem sterilem Wasser bei Raumtemperatur vermischt. Nach 24 Stunden Rühren bei Raumtemperatur mit einem Magnetrührer hatte sich eine ölig-viskose gelbliche Lösung gebildet.

### Beispiele 11 - 15: Herstellung einer erfindungsgemäßen Vorrichtung, die ein Pulver mit einer knochenwachstumsfördernden Substanz und ein Übertragungsmittel mit einer pharmazeutisch wirksamen Substanz umfasst:

Der Behälter aus Beispiel 1 wurde zunächst mit einem elastisch deformierbarem Übertragungsmittel versehen. Dann wurden mit konventionellen 10 ml Kunststoffspritzen jeweils 5 ml der Beschichtungslösungen der Beispiele 6 - 10 aufgezogen und anschließend mit den befüllten Kunststoffspritzen 4 ml der jeweiligen Wirkstofflösung auf das poröse Übertragungsmittel der Vorrichtung gespritzt. Die Wirkstofflösung wurde dabei von dem porösen Übertragungsmittel aufgesaugt.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 21: Vorrichtung
- 4, 24: Behälter
- 6, 26: Abstreifer
- 8: Übertragungsmittel
- 10, 12, 16, 30: Halterungsring
- 14: Membran
- 18: Pulver
- 27: Schlitz / Einschnitt

## Patentansprüche

1. Vorrichtung (1, 21) zur zumindest bereichsweisen Beschichtung eines medizinischen Implantats, wobei die Vorrichtung (1, 21) ein Pulver (18) beinhaltet, **dadurch gekennzeichnet, dass**
das Pulver (18) in einem Behälter (2, 24) umfassend eine Öffnung zum Einführen und Herausnehmen des medizinischen Implantats angeordnet ist und zumindest eine pharmazeutisch aktive Substanz und/oder eine knochenwachstumsfördernde Substanz umfasst, so dass beim Kontaktieren eines medizinischen Implantats das Pulver (18) auf das medizinische Implantat übertragbar ist; und
dass über dem Pulver (18) ein elastisch deformierbares Übertragungsmittel (8) angeordnet ist, mit dem eine Flüssigkeit auf das medizinische Implantat übertragbar ist, wobei die Flüssigkeit in dem Übertragungsmittel (8) enthalten ist, **dadurch gekennzeichnet, dass**
das Übertragungsmittel (8) Poren umfasst und in den Poren des Übertragungsmittels (8) die Flüssigkeit enthalten ist, wobei in der Flüssigkeit eine zweite pharmazeutisch aktive Substanz enthalten ist.

2. Vorrichtung (1, 21) nach Anspruch 1, **dadurch gekennzeichnet, dass** die Öffnung durch einen abziehbaren Deckel verschlossen ist.

3. Vorrichtung (1, 21) nach einem der Ansprüche 1 oder 2, **dadurch gekennzeichnet, dass** die Vorrichtung (1, 21) einen Abstreifer (6, 26) umfasst, der vorzugsweise im Bereich der Öffnung, insbesondere zwischen der Öffnung und dem Pulver (18) angeordnet ist.

4. Vorrichtung (1, 21) nach Anspruch 3, **dadurch gekennzeichnet, dass** der Abstreifer (6, 26) scheibenförmig ist und mindestens einen Einschnitt (27) umfasst, der die Oberseite und die Unterseite der Scheibe verbindet.

5. Vorrichtung (1, 21) nach Anspruch 3, **dadurch gekennzeichnet, dass** der Abstreifer (6, 26) als Kegelmantel (6) oder als Halbkugelfläche ausgebildet ist, wobei die Kegelspitze oder die Halbkugel in Richtung des Pulvers (18) ausgerichtet ist und vorzugsweise der Kegel oder die Halbkugel mindestens einen Einschnitt (27) enthalten, der die Oberseite und die Unterseite des Abstreifers (6, 26) verbindet.

6. Vorrichtung (1, 21) nach einem der Ansprüche 1 bis 5, **dadurch gekennzeichnet, dass** das Übertragungsmittel (8) zumindest einen Schwamm umfasst, mit der oder dem oder mit denen die Flüssigkeit auf die zu beschichtende Oberfläche des medizinischen Implantats übertragbar ist.

7. Vorrichtung (1, 21) nach einem der Ansprüche 1 bis 6, **dadurch gekennzeichnet, dass** die pharmazeutisch aktive Substanz und vorzugsweise auch die zweite pharmazeutisch aktive Substanz Antibiotika und/oder organische Antiseptika beinhaltet, so dass die zu erzeugende Beschichtung eine pharmazeutisch wirksame Dosis beinhaltet.

8. Vorrichtung (1, 21) nach einem der Ansprüche 1 bis 7, **dadurch gekennzeichnet, dass** die knochenwachstumsfördernde Substanz Calciumphosphat-Pulver, besonders bevorzugt ein Gemisch aus α- und β-Calciumphosphat, umfasst.

9. Vorrichtung (1, 21) nach einem der Ansprüche 1 bis 8, **dadurch gekennzeichnet, dass** die Vorrichtung (1, 21) einen Vakuumanschluss umfasst, der mit einer Vakuumquelle verbindbar ist und der vorzugsweise zwischen einem Abstreifer (6, 26) und dem Pulver (18) angeordnet ist.

10. Verfahren zur zumindest bereichsweisen Beschichtung eines medizinischen Implantats unter Verwendung einer Vorrichtung nach einem der Ansprüche 1 bis 9, wobei ein medizinisches Implantat mit einer zu beschichtenden Oberfläche bereitgestellt wird und
die zu beschichtende Oberfläche des medizinischen Implantats mit einem Pulver umfassend zumindest eine pharmazeutisch aktive Substanz und/oder eine knochenwachstumsfördernde Substanz kontaktiert wird, wobei durch das Kontaktieren Pulver auf die Oberfläche des medizinischen Implantats übertragen wird und nach dem Kontaktieren wenigstens ein Teil des Pulvers auf der zu beschichtenden Oberfläche haftet; **dadurch gekennzeichnet, dass**
das Pulver in dem Behälter mit der Öffnung bereitgestellt wird, wobei das medizinische Implantat vor dem Kontaktieren mit dem Pulver zur Beschichtung der zu beschichtenden Oberfläche durch die Öffnung in den Behälter eingeführt wird, in dem sich das Pulver befindet und nach der Übertragung des Pulvers auf das medizinische Implantat aus dem Behälter herausgezogen wird, und
das medizinische Implantat über das elastisch deformierbare Übertragungsmittel gestrichen wird, wobei beim Überstreichen des Übertragungsmittels eine Flüssigkeit vom Übertragungsmittel auf die zu beschichtenden Oberfläche des medizinischen Implantats übertragen wird.

11. Verfahren nach Anspruch 10, **dadurch gekennzeichnet, dass** das medizinische Implantat durch eine Membran gestoßen wird oder eine Membran geöffnet wird, bevor das medizinische Implantat mit dem Pulver kontaktiert wird, wobei die Membran das Pulver zumindest bereichsweise abdeckt, vorzugsweise die Membran das Pulver im Behälter abdichtet.

12. Verfahren nach einem der Ansprüche 10 bis 11, **dadurch gekennzeichnet, dass** ein zur Behandlungssituation passendes Pulver bereitgestellt wird und/oder ein zur Behandlungssituation passendes Antibiotikum oder Antibiotika-Gemisch in das Pulver eingebracht wird.

13. Verfahren nach einem der Ansprüche 10 bis 12, **dadurch gekennzeichnet, dass** ein Teil des übertragenen Pulvers abgestreift wird, insbesondere beim Herausziehen des medizinischen Implantats aus dem Behälter, vorzugsweise an einem dafür vorgesehenen Abstreifer.

14. Verfahren nach einem der Ansprüche 10 bis 13, **dadurch gekennzeichnet, dass** zumindest 50% der Oberfläche des medizinischen Implantats, vorzugsweise zumindest 80%, besonders bevorzugt zumindest 90% der Oberfläche des medizinischen Implantats beschichtet werden.

15. Verfahren nach einem der Ansprüche 11 bis 14, **dadurch gekennzeichnet, dass** das Pulver eingefärbt wird, so dass der beschichtete Bereich des medizinischen Implantats farblich kenntlich gemacht wird.

16. Verfahren nach Anspruch 15, **dadurch gekennzeichnet, dass** die Vollständigkeit der Beschichtung des zu beschichtenden Bereichs anhand der Färbung geprüft wird.

17. Verfahren nach einem der Ansprüche 10 bis 16, **dadurch gekennzeichnet, dass** das Verfahren so oft wiederholt wird, bis eine vollständige Beschichtung der zu beschichtenden Oberfläche des medizinischen Implantats erreicht wird.

18. Verfahren nach einem der Ansprüche 10 bis 17, **dadurch gekennzeichnet, dass** das Pulver mit einem Luftstrom aufgewirbelt wird, um eine vollständige Beschichtung des medizinischen Implantats zu erreichen.

## Claims

1. Device (1, 21) for coating at least sections of a medical implant, whereby the device (1, 21) contains a powder (18), **characterised in that**
the powder (18) is arranged in a container (2, 24) comprising an opening for inserting and taking out the medical implant, and comprises at least one pharmaceutically active substance and/or a bone growth-promoting substance such that the powder (18) contacting a medical implant can be transferred onto the medical implant; and
**in that** an elastically deformable transfer means (8) is arranged above the powder (18) by means of which a liquid can be transferred onto the medical implant, whereby the liquid is contained in the transfer means (8),
**characterised in that**
the transfer means (8) comprises pores and the pores of the transfer means (8) contain the liquid, whereby the liquid contains a second pharmaceutically active substance.

2. Device (1, 21) according to claim 1, **characterised in that**
the opening is closed by a cover that can be pulled off.

3. Device (1, 21) according to either one of the claims 1 or 2, **characterised in that**
the device (1, 21) comprises a wiper (6, 26), which preferably is arranged in the area of the opening, in particular between the opening and the powder (18).

4. Device (1, 21) according to claim 3, **characterised in that**
the wiper (6, 26) is disk-shaped and comprises at least one notch (27) that connects the top side and the bottom side of the disk.

5. Device (1, 21) according to claim 3, **characterised in that**
the wiper (6, 26) is designed in the form of the surface of a cone (6) or a hemispherical surface, whereby the tip of the cone or the hemi-sphere is oriented in the direction of the powder (18) and the cone or the hemisphere preferably contain at least one notch (27) that connects the top side and the bottom side of the wiper (6, 26).

6. Device (1, 21) according to any one of the claims 1 to 5, **characterised in that**
the transfer means (8) comprises at least one sponge by means of which the liquid can be transferred to the surface of the medical implant to be coated.

7. Device (1, 21) according to any one of the claims 1 to 6, **characterised in that**
the pharmaceutically active substance, and preferably the second pharmaceutically active substance as well, contains antibiotics and/or organic antiseptics such that the coating to be generated contains a pharmaceutically effective dose.

8. Device (1, 21) according to any one of the claims 1 to 7, **characterised in that**
the bone growth-promoting substance comprises calcium phosphate powder, particularly preferably a mixture of α- and β-calcium phosphate.

9. Device (1, 21) according to any one of the claims 1 to 8, **characterised in that**
the device (1, 21) comprises a vacuum connector that can be connected to a vacuum source and is preferred to be arranged between a wiper (6, 26) and the powder (18).

10. Method for coating at least sections of a medical implant through the use of a device according to any one of the claims 1 to 9, whereby a medical implant with a surface to be coated is provided and the surface of the medical implant to be coated is contacted to a powder comprising at least one pharmaceutically active substance and/or a bone growth-promoting substance, whereby the contacting transfers powder onto the surface of the medical implant and at least a portion of the powder adheres to the surface to be coated after the contacting;
**characterised in that**
the powder is provided in the container with the opening, whereby the medical implant is inserted through the opening into the container, in which the powder is situated, prior to being contacted to the powder for coating the surface to be coated, and is pulled out of the container after the transfer of the powder onto the medical implant, and
the medical implant is made to pass over the elastically deformable transfer means, whereby a liquid is transferred from the transfer means onto the surface of the medical implant to be coated while passing over the transfer means.

11. Method according to claim 10, **characterised in that**
the medical implant is being pushed through a membrane or a membrane is being opened before the medical implant is contacted to the powder, whereby the membrane covers at least sections of the powder, preferably the membrane seals the powder in the container.

12. Method according to either one of the claims 10 to 11, **characterised in that** a powder matching the treatment situation is provided and/or an antibiotic or mixture of antibiotics matching the treatment situation is incorporated into the powder.

13. Method according to any one of the claims 10 to 12, **characterised in that** a portion of the transferred powder is wiped off, in particular when the medical implant is pulled out of the container, preferably on a wiper design for this purpose.

14. Method according to any one of the claims 10 to 13, **characterised in that** at least 50% of the surface of the medical implant, preferably at least 80%, particularly preferably at least 90% of the surface of the medical implant are being coated.

15. Method according to any one of the claims 10 to 14, **characterised in that**
the powder is being dyed such that the coated section of the medical implant is identifiable by colour.

16. Method according to claim 15, **characterised in that**
the completeness of the coating of the section to be coated is checked by means of the colouration.

17. Method according to any one of the claims 10 to 16, **characterised in that**
the method is repeated until complete coating of the surface of the medical implant to be coated is attained.

18. Method according to any one of the claims 10 to 18, **characterised in that**
the powder is whirled up with an air flow in order to attain complete coating of the medical implant.

## Revendications

1. Dispositif (1, 21) destiné au revêtement au moins partiel d'un implant médical, le dispositif (1, 21) contenant une poudre (18), **caractérisé en ce que** la poudre (18) est disposée dans un récipient (2, 24) comprenant un orifice pour l'introduction et le retrait de l'implant médical et contient au moins une substance pharmaceutiquement active et/ou une substance stimulant la croissance osseuse de façon à ce que la poudre (18) soit transmissible sur l'implant médical lors de la mise en contact d'un implant médical ; et
qu'un moyen de transmission (8) élastiquement déformable, permettant de transmettre un liquide sur l'implant médical, est disposé au-dessus de la poudre (18), le liquide étant contenu dans le moyen de transmission (8),
**caractérisé en ce que**
le moyen de transmission (8) comprend des pores et que le liquide est contenu dans les pores du moyen de transmission (8), une deuxième substance pharmaceutiquement active étant contenue dans le liquide.

2. Dispositif (1, 21) conformément à la revendication n°1, **caractérisé en ce que** l'orifice est obturé par un couvercle amovible.

3. Dispositif (1, 21) conformément à l'une des revendications n°1 ou n°2,
**caractérisé en ce que**
le dispositif (1, 21) comprend un racleur (6, 26) qui est disposé de préférence dans la zone de l'orifice, en particulier entre l'orifice et la poudre (18).

4. Dispositif (1, 21) conformément à la revendication n°3, **caractérisé en ce que**
le racleur (6, 26) est en forme de disque et comprend au moins une entaille (27) qui relie la partie supérieure et la partie inférieure du disque.

5. Dispositif (1, 21) conformément à la revendication n°3, **caractérisé en ce que**
le racleur (6, 26) est conçu comme enveloppe conique (6) ou comme surface hémisphérique, la pointe conique ou l'hémisphère étant orienté dans la direction de la poudre (18) et le cône ou l'hémisphère contenant de préférence au moins une entaille (27) qui relie la partie supérieure et la partie inférieure du disque.

6. Dispositif (1, 21) conformément à l'une des revendications n°1 à n°5,
**caractérisé en ce que**
le moyen de transmission (8) comprend au moins une éponge avec laquelle ou lesquelles le liquide est transmissible sur la surface de l'implant médical à revêtir.

7. Dispositif (1, 21) conformément à l'une des revendications n°1 à n°6,
**caractérisé en ce que**
la substance pharmaceutiquement active et de préférence également la deuxième substance pharmaceutiquement active contient des antibiotiques et/ou des antiseptiques organiques de façon à ce que le revêtement à produire contienne une dose phamaceutiquement active.

8. Dispositif (1, 21) conformément à l'une des revendications n°1 à n°7,
**caractérisé en ce que**
la substance stimulant la croissance osseuse comprend une poudre de phosphate de calcium, en particulier de préférence un mélange de phosphate de calcium α et β.

9. Dispositif (1, 21) conformément à l'une des revendications n°1 à n°8,
**caractérisé en ce que**
le dispositif (1, 21) comprend une prise de vide qui peut être reliée à une source de vide et qui est disposée de préférence entre un racleur (6, 26) et la poudre (18).

10. Procédé destiné au revêtement au moins partiel d'un implant médical avec l'utilisation d'un dispositif conformément à l'une des revendications n°1 à n°9, un implant médical avec une surface à revêtir étant mis à disposition et la surface à revêtir de l'implant médical étant mise en contact avec une poudre comprenant au moins une substance pharmaceutiquement active et/ou une substance stimulant la croissance osseuse, la poudre étant transmise sur la surface de l'implant médical lors de la mise en contact et au moins une partie de la poudre adhérant à la surface à revêtir après la mise en contact ;
**caractérisé en ce que**
la poudre est mise à disposition dans le récipient avec l'orifice, l'implant médical étant introduit par l'orifice dans le récipient dans lequel se trouve la poudre avant la mise en contact avec la poudre destinée au revêtement de la surface à revêtir et étant retiré du récipient après la transmission de la poudre sur l'implant médical et
l'implant médical est enduit par le moyen de transmission élastiquement déformable, un liquide étant transmis du moyen de transmission sur la surface à revêtir de l'implant médical lors de l'enduction par le moyen de transmission.

11. Procédé conformément à la revendication n°10, **caractérisé en ce que**
l'implant médical est poussé par une membrane ou qu'une membrane est ouverte avant que l'implant médical soit mis en contact avec la poudre, la membrane recouvrant la poudre au moins partiellement, de préférence la membrane colmatant la poudre dans le récipient.

12. Procédé conformément à l'une des revendications n°10 à n°11, **caractérisé en ce**
**qu'**une poudre appropriée à la situation de traitement est mise à disposition et/ou qu'un antibiotique ou mélange d'antibiotiques approprié à la situation de traitement est introduit dans la poudre.

13. Procédé conformément à l'une des revendications n°10 à n°12, **caractérisé en ce**
**qu'**une partie de la poudre transmise est raclée, en particulier lors du retrait de l'implant médical du récipient, de préférence sur un racleur prévu à cet effet.

14. Procédé conformément à l'une des revendications n°10 à n°13, **caractérisé en ce**
**qu'**au moins 50% de la surface de l'implant médical, de préférence au moins 80%, en particulier de préférence au moins 90% de la surface de l'implant médical sont recouverts.

15. Procédé conformément à l'une des revendications n°11 à n°14, **caractérisé en ce que**
la poudre est colorée de façon à ce que la zone revêtue de l'implant médical puisse être identifiable par la couleur.

16. Procédé conformément à la revendication n°15, **caractérisé en ce que** l'intégralité du revêtement de la zone à revêtir est vérifiée au moyen de la coloration.

17. Procédé conformément à l'une des revendications n°10 à n°16, **caractérisé en ce que**
le procédé est répété jusqu'à ce qu'un revêtement intégral de la surface à revêtir de l'implant médical soit obtenu.

18. Procédé conformément à l'une des revendications n°10 à n°17, **caractérisé en ce que**
la poudre est mise en tourbillons par un courant d'air pour obtenir un revêtement intégral de l'implant médical.
